# FASCICULE DE BREVET EUROPEEN

(11) **EP 1 430 920 B1**
(45) Date de publication et mention de la délivrance du brevet: **25.07.2007**
(21) Numéro de dépôt: 03356203.4
(22) Date de dépôt: 18.12.2003
(51) Int. Cl.: A61M 1/34, A61M 1/36

(54) **Dispositif pour le traitement extracorporel du sang par anticoagulation au citrate**
Vorrichtung zur extrakorporalen Blutbehandlung durch Zitratantikoagulation
Device for extracorporeal blood treatment by citrate anticoagulation

(30) Priorité: 20.12.2002 FR 0216228
(43) Date de publication de la demande: 23.06.2004
(73) Titulaire: Gambro Lundia AB, 22 643 Lund (SE)
(72) Inventeur: Bene, Bernard, 69540 Irigny (FR)
(74) Mandataire: Sutto, Luca

(56) Documents cités:
- EP-A- 0 578 175
- EP-A- 0 829 265
- WO-A-00/64456
- US-A- 4 500 309
- US-A- 5 344 568

## Description

La présente invention a pour objet un dispositif de traitement de sang par circulation extracorporelle.

La réalisation d'une séance d'hémodialyse nécessite d'anticoaguler le sang circulant dans la ligne de circulation extra-corporelle afin d'éviter la coagulation du sang au contact des matériaux synthétiques (lignes de circulation, fibres du dialyseur). Le plus souvent, cette anticoagulation est réalisée en utilisant de l'héparine, connue pour ses propriétés anticoagulantes. L'héparine est injectée en pré-infusion dans la ligne artérielle du dispositif, celle-ci est alors présente dans tout le circuit extracorporel du sang, depuis la pré-infusion, jusque la réinjection du sang dans le patient. Dès lors, le patient se voit administré, par le retour sanguin, des doses d'héparine. Et, même s'il est nécessaire d'obtenir une prévention de la coagulation dans la ligne de circulation extra-corporelle, dans certains cas le risque de saignement du patient peut présenter un danger. Il s'agit en particulier des patients à fort risque hémorragique (par exemple dans les jours qui suivent une intervention chirurgicale majeure) ou encore de patients ayant une hypersensibilité à l'héparine. Dès lors, le traitement d'anticoagulation par héparine peut s'avérer dangereux pour le patient du fait de l'injection d'héparine via le retour sanguin au patient.

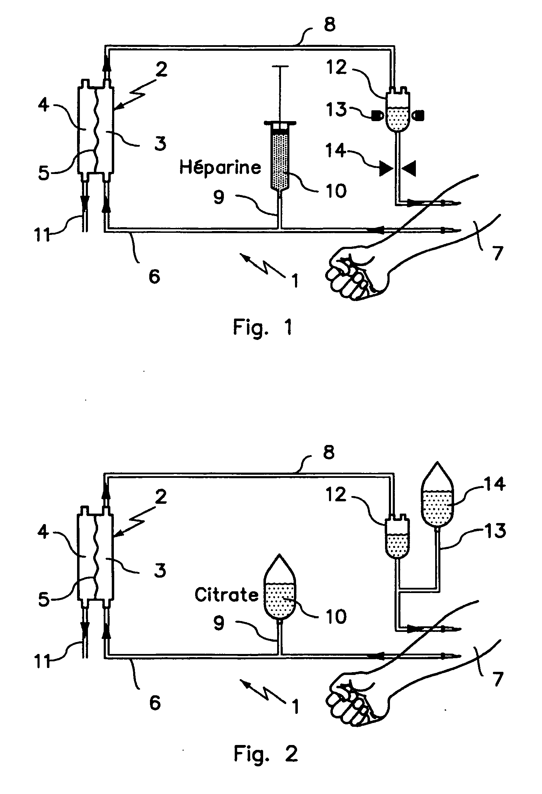

La figure 1 représente un dispositif de traitement de sang par circulation extracorporelle 1 de l'état de l'art comprenant un filtre 2 ayant une première chambre 3 et une deuxième chambre 4 séparées par une membrane semi-perméable 5. Le dispositif comprend une ligne artérielle 6 reliée à la première chambre 3 du filtre pour la circulation de sang prélevé d'un patient 7, et une ligne veineuse 8 en sortie de la première chambre du filtre. Une canalisation 9 de pré-infusion est reliée à un conteneur 10 comportant une solution d'héparine et est branchée sur la ligne artérielle 6, la solution ayant pour fonction d'empêcher la coagulation du sang à l'extérieur du corps du patient, une canalisation de purge 11 en sortie de la deuxième chambre 4 du filtre. Un séparateur d'air 12 est branché sur la ligne veineuse 8. Le séparateur d'air 12 comprend également un détecteur d'air 13, et un clamp 14 est placé sur la ligne veineuse 8 en aval du séparateur d'air 12, c'est-à-dire en aval dans le sens de la circulation du sang dans la ligne veineuse 8. Le clamp permet d'arrêter le flux sanguin en cas de détection de bulles d'air par le détecteur 13 avant que le sang ne retourne au patient 7.

Ainsi, la solution d'héparine, comme mentionné plus haut, agit contre la coagulation du sang à la fois dans la totalité du dispositif 1 et dans le corps du patient. Dès lors, le retour et l'injection d'héparine dans le corps du patient 7 peut s'avérer dangereux.

La demande de brevet PCT/EP00/03583 (WO 0064456) divulgue un dispositif selon le préambule de la revendication 1. Elle concerne un fluide d'infusion de substitution, en particulier pour l'utilisation dans l'hémofiltration de sang, et une solution d'anticoagulant au citrate pour l'anticoagulation régionale. Afin d'éviter la coagulation du sang, lors d'une séance d'hémodialyse, il est connu que les ions citrate peuvent être utilisés comme anticoagulant. Les ions citrate, ajoutés au sang dans le circuit extracorporel avant qu'ils ne pénètrent dans le rein artificiel, sont actifs en tant qu'anticoagulants. Ainsi, le risque de complications de saignement dues à l'anticoagulation systématique est évité. Durant l'hémofiltration, une partie des ions citrate passent au travers du rein artificiel. Les ions citrate sont actifs en tant qu'anticoagulants seulement dans le circuit extracorporel, car lorsqu'ils atteignent la circulation systémique du patient, ils sont rapidement métabolisés en ions bicarbonate. La demande de brevet PCT/EP00/03583 a pour objet une solution d'une certaine composition.

La figure 2 représente un dispositif de traitement de sang par circulation extracorporelle 1 décrit dans la demande de brevet PCT/EP00/03583. Ce dispositif 1 comprend un filtre 2 ayant une première chambre 3 et une deuxième chambre 4 séparées par une membrane semi-perméable 5. Le dispositif comprend une ligne artérielle 6 reliée à la première chambre 3 du filtre pour la circulation de sang prélevé d'un patient 7, et une ligne veineuse 8 en sortie de la première chambre du filtre. Une canalisation 9 de pré-infusion contenant un anti-coagulant, le citrate de trisodium, est reliée à un conteneur 10 comportant une solution d'ions citrate branchée sur la ligne artérielle 6, la solution ayant pour fonction d'empêcher la coagulation du sang à l'extérieur du corps du patient. Une canalisation de purge 11 est branchée en sortie de la deuxième chambre 4 du filtre et un séparateur d'air 12 est branché sur la ligne veineuse 8.

La ligne veineuse 8 comporte, en plus du piège à bulles 12, une canalisation 13 reliée à un conteneur 14 comportant une solution de rétablissement de l'équilibre ionique du sang. En effet, la solution de citrate rend le sang anti-coagulable en le décalcifiant en amont du filtre. Cette canalisation 13 est branchée en aval du piège à bulles 12, c'est-à-dire en aval dans le sens de la circulation du sang dans la ligne veineuse 8 avant le retour du sang au patient 7. En effet, pour rétablir une hémostase correcte, on doit rétablir l'équilibre ionique du sang -en le recalcifiant notamment- en sortie du filtre.

L'enseignement de ce document comprend un dispositif de sécurité permettant de détecter d'éventuelles bulles d'air présentes au début de la ligne veineuse. En revanche, le dispositif décrit ne prévoit pas de moyen de sécurité permettant de détecter et de piéger d'éventuelles bulles d'air dans la ligne de post-infusion branchée sur la ligne veineuse, et un retour d'air dans la circulation systémique du patient, tout à fait dangereux pour celui-ci, est possible.

Pour tenter de résoudre ce problème, il a été envisagé par l'homme du métier de brancher la post-infusion contenant une solution d'infusion de substitution directement sur le piège à bulles de la ligne veineuse et non en aval de celui-ci. Aussi, le détecteur de bulles a été placé sur ce piège à bulles. De cette manière, un unique dispositif permet à la fois de séparer et de détecter les bulles d'air venant d'une part du liquide sanguin extrait du filtre et d'autre part du liquide de post-infusion.

Cependant, lors d'essais menés avec cette disposition, il a été constaté, surtout lors d'un fonctionnement long tel qu'en unité de soins intensifs à faible débit sanguin, que si le problème des bulles d'air est résolu pour les deux liquides, il se pose un nouveau problème : celui de la coagulation au sein du piège à bulles : en effet, le liquide sanguin extrait du filtre et arrivant dans le piège à bulles comporte un liquide anticoagulant injecté en pré-infusion dans le sang du patient lors de son passage dans la ligne artérielle. De plus, le piège à bulles reçoit aussi une solution d'infusion de substitution ayant pour effet de neutraliser l'effet anticoagulant de la solution de pré-infusion. La structure du piège à bulles est telle que d'une part le liquide passant à l'intérieur de celui-ci est en contact avec l'air et le risque de coagulation est présent; et que d'autre part le piège à bulles comportant un diamètre plus grand que le diamètre de la ligne veineuse, le débit de fluide s'en trouve d'autant diminué et le phénomène de coagulation s'en trouve d'autant favorisé.

Il est également connu dans l'art antérieur le brevet US-5,330,425 (Utterberg) qui concerne seulement l'injection dans la ligne veineuse de médicaments ou similaires. Dans ce brevet, on craint que certains médicaments restent piégés dans la chambre veineuse latéralement où la vélocité du sang est réduite. Avec une machine d'hémodialyse ayant un ensemble détecteur d'air - clamp de ligne monté sur la ligne veineuse typiquement au moins 3,08 centimètres (2 pouces) sous la chambre veineuse, il est possible de réaliser une ligne à sang veineuse où un site d'injection est placé sur le tube en aval de la chambre veineuse, déjà en amont du détecteur d'air - clamp de ligne.

Un autre état de la technique est le brevet US-4,490,135 (Troutner), il concerne un système d'hémodialyse à aiguille unique ayant deux détecteurs d'air sur la ligne veineuse.

Dès lors se pose le double problème de détecter les bulles dans chacun des deux fluides par un dispositif de structure simple et d'empêcher la coagulation du sang dans la totalité du circuit extracorporel.

Le but de la présente invention consiste à réaliser un dispositif qui permet de résoudre ce problème.

Pour atteindre ce but on prévoit, conformément à l'invention, un dispositif de traitement de sang par circulation extracorporelle (101) selon la revendication 1.

D'autres avantages et caractéristiques de l'invention apparaîtront à la lecture de la description qui suit.

On se reportera aux dessins annexés sur lesquels :
la figure 1 représente l'état de l'art concernant une installation de traitement extracorporel sanguin avec prévention de l'anticoagulation par héparine;
la figure 2 représente l'état de l'art concernant une installation de traitement extracorporel sanguin avec prévention de l'anticoagulation par citrate ;
la figure 3 représente le dispositif de traitement de sang par circulation extracorporelle selon l'invention ;
la figure 4 représente une ligne disposable pour le traitement de sang par circulation extracorporelle.

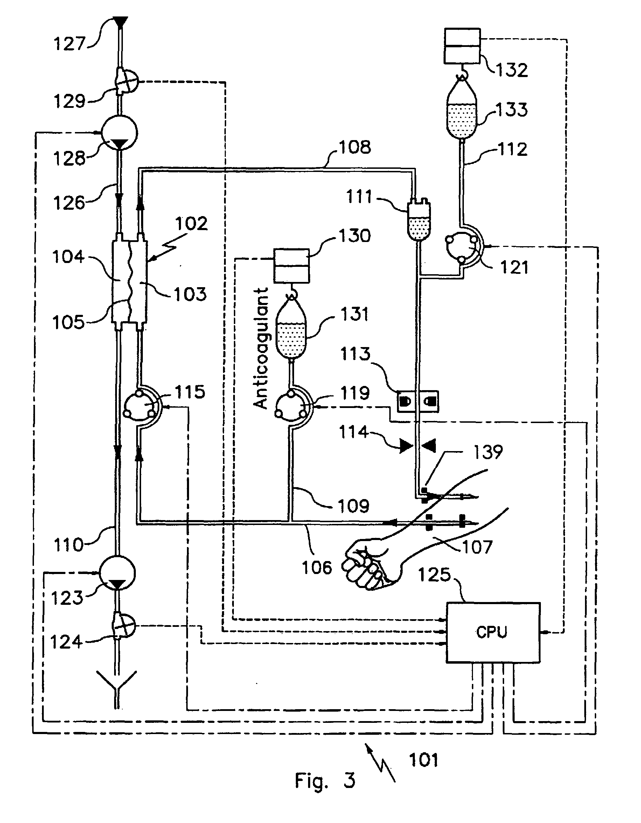

La figure 3 représente le dispositif de traitement extracorporel sanguin selon l'invention, propre à effectuer un traitement de dialyse. Le dispositif de traitement de sang par circulation extracorporelle 101 selon l'invention comprend un filtre 102 ayant une première chambre 103 et une deuxième chambre 104 séparées par une membrane semi-perméable 105. En fonction de la membrane, le filtre (102) peut être un hémofiltre, un plasmafiltre, un dialyseur, ou un filtre d'une autre nature encore. Au niveau de la membrane, les membranes utilisées sont des membranes en fibres creuses ou des membranes à plaques ou en gaine. Une ligne artérielle 106 est reliée à la première chambre 103 du filtre pour la circulation de sang prélevé d'un patient 107. Une ligne veineuse 108 est reliée en sortie de la première chambre 103 du filtre. Une canalisation 109 de pré-infusion d'une substance d'anticoagulation régionale est branchée sur la ligne artérielle 106. Une canalisation de purge 110 est reliée en sortie de la deuxième chambre 104 du filtre. Un séparateur d'air 111 est placé sur la ligne veineuse 108 et une ligne de post-infusion 112 d'une solution rétablissant au moins partiellement l'équilibre ionique du sang est placée en aval -c'est-à-dire en aval dans le sens de la circulation du sang dans la ligne veineuse 108- du séparateur d'air 111. Enfin, un détecteur d'air 113 est placé en aval de la ligne de post-infusion 112.

Aucun autre séparateur d'air n'est inséré en aval du détecteur d'air 113. Aussi, le séparateur d'air 111 peut être le seul séparateur d'air présent dans la ligne veineuse 108 et ne contient pas de détecteur d'air.

De cette manière, les bulles d'air éventuellement amenées par la post-infusion restent séparables avec une ligne comportant un seul séparateur d'air, sans entraîner une coagulation non souhaitée dans le circuit extracorporel, en particulier dans le séparateur d'air.

La ligne veineuse 108 comprend une canalisation pouvant recevoir un moyen d'occlusion 114 en aval du détecteur d'air. Le moyen d'occlusion 114 comprend un clamp ou une vanne d'une autre structure. Ce moyen permet, en cas de détection d'air, d'arrêter le retour sanguin vers le patient afin de pouvoir éliminer les bulles d'air détectées.

La ligne de post-infusion 112 est montée immédiatement en amont du détecteur d'air 113.

Aucun raccord de ligne ou aucune zone d'injection ne sera placée sur la ligne veineuse 108 entre le détecteur d'air 113 et le raccord 139 de ligne veineuse au patient. Le raccord est apte à se connecter au dispositif d'accès vasculaire du patient (non représenté sur les figures) qui lui-même peut être par exemple une aiguille ou un cathéter.

De plus, le détecteur d'air 113 est connecté le plus proche possible du raccord de la ligne veineuse (108) au patient, par exemple à 2 centimètres, juste en amont du dispositif d'accès vasculaire.

Ainsi, le pouvoir anticoagulant de la solution de pré-infusion est utilisé dans la plus grande partie possible du dispositif.

Le séparateur d'air 111 comprend un piège à bulles.

La solution d'anticoagulation régionale contient des ions citrate, par exemple du citrate trisodique dihydraté ou une solution d'Anticoagulant Citrate Dextrose dite « ACD » comportant du citrate trisodique dihydraté et de l'acide citrique monohydraté.

La solution rétablissant au moins partiellement l'équilibre ionique du sang contient des ions calcium. Cette solution peut contenir une solution isotonique comprenant du chlorure de calcium, de potassium et de magnésium.

La ligne artérielle 106 est opérationnellement associée à un premier moyen de réglage de débit sanguin 115.

La ligne de pré-infusion 109 est connectée à la ligne artérielle en amont de tout moyen de réglage du débit de liquide présent sur la ligne artérielle 108 dans le sens de la circulation du sang, par exemple en amont de la pompe à sang 115.

La ligne de pré-infusion 109 est connectée à la ligne artérielle 106 le plus proche possible du raccord de la ligne artérielle au patient.

Ainsi, le pouvoir anti-coagulant de la solution de pré-infusion est utilisé le plus tôt possible dans le circuit extracorporel.

La ligne de pré-infusion est opérationnellement associée à un deuxième moyen de réglage du débit de liquide 119.

La ligne de post-infusion 112 est opérationnellement associée à un troisième moyen de réglage du débit de liquide 121.

La ligne de purge 110 en sortie de la deuxième chambre 104 du filtre 102 comporte un quatrième moyen de réglage du débit de liquide 123 et un premier moyen de mesure du débit de liquide 124.

Une unité de calcul et de commande (CPU) 125 permet de recevoir les signaux émis par un premier moyen de mesure de débit 124 et de contrôler un ou plusieurs moyens de réglage de débit (115, 119, 121, 123) afin de commander un mode de fonctionnement d'hémofiltration.

Le dispositif de traitement de sang par circulation extracorporelle 101 comporte une ligne d'entrée 126 à la deuxième chambre 104 du filtre 102 reliant une source de liquide de dialyse 127, par exemple un conteneur de liquide stérilisé ou un dispositif de préparation de liquide en ligne.

Les ions citrate se lient naturellement aux ions calcium : c'est pourquoi le liquide de dialyse ne comporte pas d'ions calcium : ceci permet d'éviter la réaction du citrate contenu dans la pré-infusion avec du calcium qui serait contenu dans le liquide de dialyse. Le citrate contenu dans la pré-infusion réagit de ce fait uniquement avec le calcium contenu dans le sang et le pouvoir anticoagulant du citrate n'est pas atténué par le liquide de dialyse.

La ligne d'entrée 126 comporte un cinquième moyen de réglage du débit de liquide 128 et un deuxième moyen de mesure du débit de liquide 129.

Une unité de calcul et de commande (CPU) 125 permet de recevoir les signaux émis par un ou plusieurs moyens de mesure de débit de liquide (124, 129) et de contrôler un ou plusieurs moyens de réglage de débit de liquide (115, 119, 121, 123, 128) afin de commander un mode de fonctionnement d'hémofiltration ou d'hémodiafiltration.

Chaque moyen de réglage du débit de liquide (115, 119, 121, 123, 128) comprend une pompe ou une vanne. Chaque moyen de mesure du débit de liquide (124, 129) comprend un débitmètre.

Une première balance 130 permet de peser un premier réservoir 131 relié à la ligne de pré-infusion 109 et contenant le liquide de pré-infusion ; et une deuxième balance 132 permet de peser un deuxième réservoir 133 relié à la ligne de post-infusion 112 et contenant le liquide de post-infusion.

L'unité de calcul et de commande 125 permet de recevoir les signaux émis par au moins une des deux balances (130, 132). Elle contrôle un ou plusieurs moyens de réglage de débit de liquide (119, 121) en calculant périodiquement le débit réel ou un paramètre fonction du débit réel.

Ainsi, les quantités d'anticoagulant et de solution pour rétablir l'équilibre ionique peuvent être connues et contrôlées pendant le traitement. Connaissant lesdits poids, l'unité de commande et de contrôle (125) est en mesure de commander directement les moyens de réglage de débit de liquide afin d'obtenir une quantité souhaitée de solution de pré-infusion et/ou de solution de post-infusion dans le circuit extracorporel.

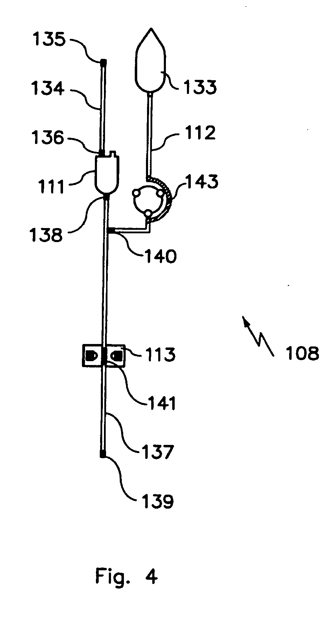

La figure 4 représente la ligne veineuse disposable 108 pour l'utilisation dans un circuit de circulation extracorporelle de sang ayant subi une pré-infusion d'anticoagulant comprenant une première canalisation 134 ayant un premier raccord 135 pour brancher un filtre et un deuxième raccord 136. Un séparateur d'air 111 est branché au deuxième raccord 136 en aval -c'est-à-dire en aval dans le sens de la circulation du sang- de la première canalisation 134. La ligne veineuse disposable 108 comprend également une deuxième canalisation 137 ayant un troisième raccord 138 pour brancher la sortie du séparateur d'air 111 et un quatrième raccord 139 pour brancher l'accès sanguin d'un patient. Un cinquième raccord 140 est placé pour brancher une ligne de post-infusion 112 d'une solution rétablissant au moins partiellement l'équilibre ionique du sang, le cinquième raccord 140 étant en communication de fluide avec la deuxième canalisation 137. La ligne comporte en outre une première partie 141 de la deuxième canalisation 137 capable de coopérer avec le détecteur d'air 113 et placée en aval du cinquième raccord 140.

La première partie 141 est marquée optiquement, par exemple par des couleurs différentes, par un relief, par un tuyau différencié au niveau du matériel, de la forme ou de la dimension par rapport au reste de la ligne.

La ligne de post-infusion 112 est branchée sur le cinquième raccord 140 pour infuser la solution rétablissant au moins partiellement l'équilibre ionique du sang.

Le cinquième raccord 140 de la ligne veineuse sera branché sur la deuxième canalisation 137 le plus proche possible du quatrième raccord 139.

Aucun raccord de ligne ou aucune zone d'injection n'est placé entre le cinquième raccord 140 de la ligne de post-infusion et le quatrième raccord 139 de la ligne veineuse.

Ainsi le pouvoir anticoagulant de la solution de pré-infusion et utilisé le plus tard possible et sur la plus grande partie possible du circuit extracorporel sanguin du patient.

Le cinquième raccord 140 placé sur la deuxième canalisation 137 peut être constitué par un raccord amovible. Alternativement, le connecteur 140 peut comprendre une portion terminale du tuyau 112 fixé à la ligne 137.

Aucun autre séparateur d'air n'est inséré en aval de la première partie 141 de la deuxième canalisation 137 connectable au détecteur d'air 113.

Le séparateur d'air 111 peut être le seul séparateur d'air présent dans la ligne veineuse disposable 108.

Le séparateur d'air 111 comprend un piège à bulles.

La solution rétablissant au moins partiellement l'équilibre ionique du sang contient des ions citrate.

La ligne de post-infusion 112 comporte une deuxième partie 143 connectable à un moyen de réglage de débit de la ligne de perfusion, ladite deuxième partie 143 étant maintenue en forme de U par le biais d'une barrette fixant deux points de la ligne pour faciliter le couplage avec la pompe.

La deuxième partie 143 est marquée optiquement, par exemple par des couleurs différentes, par un relief, par un tuyau différencié au niveau du matériel, de la forme ou de la dimension par rapport au reste de la ligne.

Le premier raccord 135 de la première canalisation 134 est indifféremment fixe ou amovible.

Le deuxième raccord 136 de la première canalisation 134 et le troisième raccord 138 de la deuxième canalisation 137 sont fixes.

Le quatrième raccord 139 de la deuxième canalisation 137 est indifféremment fixe ou amovible.

La présente invention apporte de nombreux avantages. En effet, les bulles d'air éventuellement amenées par la post-infusion restent détectables avec une ligne comportant un seul et unique piège à bulles : le dispositif a donc une structure simple et peu coûteuse.

De plus, la mise en oeuvre dudit dispositif, de ladite ligne et dudit procédé n'entraîne pas de coagulation non souhaitée dans le circuit extracorporel, en particulier dans le piège à bulles où le contact de l'air avec le sang est possible et où la vitesse de déplacement est inférieure au reste de la ligne veineuse du fait du diamètre plus grand du piège à bulles que celui du reste de la ligne. Ainsi on obtient une prévention efficace de l'administration d'air au patient.

Aussi, le pouvoir anticoagulant de la solution de pré-infusion est utilisé dans la plus grande partie possible du dispositif car la pré-infusion d'anticoagulant est injectée le plus possible en amont dans le circuit extracorporel et la post-infusion de solution de rétablissement d'équilibre ionique est branchée le plus proche possible de l'accès sanguin du patient, c'est-à-dire le plus possible en aval de la ligne veineuse. Ce branchement empêche la coagulation sur toute la portion de ligne où le besoin d'anticoagulation se fait sentir, particulièrement où un contact du sang avec l'air est réalisable. La disposition de la ligne veineuse est d'autant plus avantageuse qu'il résulte de la localisation du site d'administration de la solution de neutralisation qu'à aucun endroit le sang -ne comportant plus de substance anticoagulante active- ne peut être en contact avec l'air.

En outre, l'invention est très avantageuse lors d'un fonctionnement long d'un dispositif tel qu'en unité de soins intensifs à faible débit sanguin, c'est-à-dire dans un traitement aigu à faible débit et longue durée.

Il a été constaté que les valeurs de dialysance mesurées lors du traitement extracorporel selon l'invention restent aussi stables qu'avec un anticoagulant du type héparine réinjecté au patient.

Aussi, le résultat d'anticoagulation est très avantageux car le temps de coagulation est augmenté quatre fois entre la perfusion d'anticoagulant et la perfusion de solution de neutralisation par rapport au sang circulant dans le reste du circuit.

Enfin, à la fin de la séance, le temps de compression nécessaire à l'hémostase de la fistule est réduit par rapport à un traitement avec administration d'héparine car le sang réinjecté au patient a retrouvé son pouvoir coagulant normal.

## Revendications

1. Dispositif de traitement de sang par circulation extracorporelle (101) comprenant :
- un filtre (102) ayant une première (103) et une deuxième (104) chambres séparées par une membrane semi-perméable (105),
- une ligne artérielle (106) reliée à la première chambre (103) du filtre (102),
- une ligne veineuse (108) en sortie de la première chambre (103) du filtre (102), la ligne veineuse ayant un raccord (139) au patient,
- une canalisation de pré-infusion (109) d'une substance d'anticoagulation locale branchée sur la ligne artérielle (106),
- une canalisation de purge (110) en sortie de la deuxième chambre (104) du filtre (102),
- un séparateur d'air (111) sur la ligne veineuse (108),
- une ligne de post-infusion (112) d'une solution rétablissant au moins partiellement l'équilibre ionique du sang, la ligne de post-infusion étant en aval du séparateur d'air (111),
- un réservoir (133) relié à la ligne de post-infusion (112) contenant une solution rétablissant au moins partiellement l'équilibre ionique du sang,
**caractérisé en ce que:**
- il comprend un détecteur d'air (113) immédiatement en aval de la ligne de post-infusion (112),
- aucun raccord de ligne ou aucune zone d'injection n'est placé sur la ligne veineuse (108) entre le détecteur d'air (113) et le raccord (139) de ligne veineuse au patient.

2. Dispositif de traitement de sang par circulation extracorporelle selon la revendication 1 **caractérisé en ce qu**'aucun autre séparateur d'air n'est inséré en aval du détecteur d'air (113).

3. Dispositif de traitement de sang par circulation extracorporelle selon la revendication 1 ou 2 **caractérisé en ce que** ledit séparateur d'air (111) est le seul séparateur d'air présent dans la ligne veineuse (108) et ne contient pas de détecteur d'air.

4. Dispositif de traitement de sang par circulation extracorporelle selon une des revendications précédentes **caractérisé en ce que** la ligne veineuse (108) comprend une canalisation pouvant recevoir un moyen d'occlusion (114) en aval du détecteur d'air.

5. Dispositif de traitement de sang par circulation extracorporelle selon la revendication 4 **caractérisé en ce que** le moyen d'occlusion (114) est un clamp.

6. Dispositif de traitement de sang par circulation extracorporelle selon la revendication 1 **caractérisé en ce que** le détecteur d'air (113) est placé le plus proche possible du raccord de la ligne veineuse (108) au patient.

7. Dispositif de traitement de sang par circulation extracorporelle selon une des revendications précédentes **caractérisé en ce que** le séparateur d'air (111) comprend un piège à bulles.

8. Dispositif de traitement de sang par circulation extracorporelle selon une des revendications précédentes **caractérisé en ce que** la solution d'anticoagulation régionale contient des ions citrate.

9. Dispositif de traitement de sang par circulation extracorporelle selon la revendication 8 **caractérisé en ce que** la solution d'anticoagulation régionale contient une solution de citrate trisodique dihydraté.

10. Dispositif de traitement de sang par circulation extracorporelle selon la revendication 8 **caractérisé en ce que** la solution d'anticoagulation régionale contient une solution d'Anticoagulant Citrate Dextrose dite « ACD » comprenant du citrate trisodique dihydraté et de l'acide citrique monohydraté.

11. Dispositif de traitement de sang par circulation extracorporelle selon une des revendications précédentes **caractérisé en ce que** la solution rétablissant au moins partiellement l'équilibre ionique du sang contient des ions calcium.

12. Dispositif de traitement de sang par circulation extracorporelle selon la revendication 8 **caractérisé en ce que** la solution rétablissant au moins partiellement l'équilibre ionique du sang contient une solution isotonique comprenant du chlorure de calcium, de potassium et de magnésium.

13. Dispositif de traitement de sang par circulation extracorporelle selon une des revendications précédentes **caractérisé en ce que** la ligne artérielle (106) est opérationnellement associée à un premier moyen de réglage de débit sanguin (115).

14. Dispositif de traitement de sang par circulation extracorporelle selon une quelconque des revendications précédentes **caractérisé en ce que** la ligne de pré-infusion (109) est connectée à la ligne artérielle en amont de tout moyen de réglage de débit de liquide (115) présent sur la ligne artérielle (108).

15. Dispositif de traitement de sang par circulation extracorporelle selon une quelconque des revendications précédentes **caractérisé en ce que** la ligne de pré-infusion (109) est connectée à la ligne artérielle (106) le plus proche possible du raccord de la ligne artérielle au patient.

16. Dispositif de traitement de sang par circulation extracorporelle selon une des revendications précédentes **caractérisé en ce que** la ligne de pré-infusion (109) est opérationnellement associée à un deuxième moyen de réglage du débit de liquide (119).

17. Dispositif de traitement de sang par circulation extracorporelle selon une des revendications précédentes **caractérisé en ce que** la ligne de post-infusion (112) est opérationnellement associée à un troisième moyen de réglage du débit de liquide (121).

18. Dispositif de traitement de sang par circulation extracorporelle selon une des revendications précédentes **caractérisé en ce que** la ligne de purge (110) en sortie de la deuxième chambre (104) du filtre (102) comporte un quatrième moyen de réglage du débit de liquide (123) et un premier moyen de mesure du débit de liquide (124).

19. Dispositif de traitement de sang par circulation extracorporelle selon une ou plusieurs des revendications 13 à 18 **caractérisé en ce qu**'il comprend une unité de calcul et de commande (125) pour recevoir les signaux émis par un moyen de mesure de débit de liquide (124) et pour contrôler un ou plusieurs moyens de réglage de débit de liquide (115, 119, 121, 123) afin de commander un mode de fonctionnement d'hémofiltration.

20. Dispositif de traitement de sang par circulation extracorporelle selon une ou plusieurs des revendications 13 à 19 **caractérisé en ce qu'**il comporte une ligne d'entrée (126) à la deuxième chambre (104) du filtre (102) reliant une source de liquide de dialyse (127).

21. Dispositif de traitement de sang par circulation extracorporelle selon la revendication 20 **caractérisé en ce que** le liquide de dialyse ne comporte pas d'ions calcium.

22. Dispositif de traitement de sang par circulation extracorporelle selon la revendication 20 ou 21 **caractérisé en ce que** la ligne d'entrée (126) comporte un cinquième moyen de réglage du débit de liquide (128) et un deuxième moyen de mesure du débit de liquide (129).

23. Dispositif de traitement de sang par circulation extracorporelle selon la revendication 20, 21 ou 22 **caractérisé en ce qu**'il comporte une unité de calcul et de commande (125) pour recevoir les signaux émis par un ou plusieurs moyens de mesure de débit de liquide (124, 129) et pour contrôler un ou plusieurs moyens de réglage de débit de liquide (115, 119, 121, 123, 128) afin de commander un mode de fonctionnement d'hémofiltration ou d'hémodiafiltration.

24. Dispositif de traitement de sang par circulation extracorporelle selon une des revendications 13 à 23 **caractérisé en ce que** chaque moyen de réglage du débit de liquide (115, 119, 121, 123, 128) comprend une pompe ou une vanne.

25. Dispositif de traitement de sang par circulation extracorporelle selon une des revendications 11 à 20 **caractérisé en ce que** chaque moyen de mesure du débit de liquide (124, 129) comprend un débitmètre.

26. Dispositif de traitement de sang par circulation extracorporelle selon une des revendications 13 à 25 **caractérisé en ce qu**'il comporte une première balance (130) pour peser un premier réservoir (131) relié à la ligne de pré-infusion (109) et contenant le liquide de pré-infusion.

27. Dispositif de traitement de sang par circulation extracorporelle selon une des revendications 13 à 26 **caractérisé en ce qu**'il comporte une deuxième balance (132) pour peser le deuxième réservoir (133) relié à la ligne de post-infusion (112) et contenant le liquide de post-infusion.

28. Dispositif de traitement de sang par circulation extracorporelle selon une des revendications 26 ou 27 **caractérisé en ce qu**'il comporte une unité de calcul et de commande (125) pour recevoir les signaux émis par au moins une des deux balances (130, 132) et pour contrôler un ou plusieurs moyens de réglage de débit de liquide (115, 119, 121, 123, 128).

## Claims

1. A blood treatment device by way of extracorporeal circulation (101) comprising:
- a filter (102) having a first (103) and a second (104) compartment separated by a semipermeable membrane (105),
- an arterial line (106) connected to the first compartment (103) of the filter (102),
- a venous line (108) getting out of the first compartment (103) of the filter (102), the venous line having a connection (139) to the patient,
- a pre-infusion channel (109) for a local anticoagulation substance connected to the arterial line (106),
- a drain channel (110) getting out of the second compartment (104) of the filter (102),
- an air separator (111) on the venous line (108),
- a post-infusion line (112) for a solution at least partially reestablishing blood ion balance, the post-infusion line being located downstream from the air separator (111),
- a container (133) connected to the post-infusion line (112) and containing a solution at least partially reestablishing blood ion balance,
**characterized in that**:
- it comprises an air detector (113) immediately downstream from the post-infusion line (112),
- no line connection or injection zone is located on the venous line (108) between the air detector (113) and the connection (139) of the venous line to the patient.

2. Blood treatment device by way of extracorporeal circulation according to claim 1, **characterized in that** no other air separator is installed downstream from the air detector (113).

3. Blood treatment device by way of extracorporeal circulation according to claim 1 or 2, **characterized in that** said air separator (111) is the only air separator present in the venous line (108) and contains no air detector.

4. Blood treatment device by way of extracorporeal circulation according to one of the preceding claims, **characterized in that** the venous line (108) comprises a channel that is able to receive an obstruction means (114) downstream from the air detector.

5. Blood treatment device by way of extracorporeal circulation according to claim 4, **characterized in that** the obstruction means (114) is a clamp.

6. Blood treatment device by way of extracorporeal circulation according to claim 1, **characterized in that** the air detector (113) is placed as close as possible to the connection of the venous line (108) to the patient.

7. Blood treatment device by way of extracorporeal circulation according to one of the preceding claims, **characterized in that** the air separator (111) comprises a bubble trap.

8. Blood treatment device by way of extracorporeal circulation according to one of the preceding claims, **characterized in that** the local anticoagulation solution contains citrate ions.

9. Blood treatment device by way of extracorporeal circulation according to claim 8, **characterized in that** the local anticoagulation solution contains a solution of trisodium citrate dihydrate.

10. Blood treatment device by way of extracorporeal circulation according to claim 8, **characterized in that** the local anticoagulation solution contains a solution of Anticoagulant Citrate Dextrose known as "ACD" comprising trisodium citrate dihydrate and citric acid monohydrate.

11. Blood treatment device by way of extracorporeal circulation according to one of the preceding claims, **characterized in that** the solution at least partially reestablishing blood ion balance contains calcium ions.

12. Blood treatment device by way of extracorporeal circulation according to claim 8, **characterized in that** the solution at least partially reestablishing blood ion balance contains an isotonic solution comprising calcium, potassium and magnesium chloride.

13. Blood treatment device by way of extracorporeal circulation according to one of the preceding claims, **characterized in that** the arterial line (106) is operatively associated to a first means for adjusting blood flow rate (115).

14. Blood treatment device by way of extracorporeal circulation according to any one of the preceding claims, **characterized in that** the pre-infusion line (109) is connected to the arterial line upstream from any means for adjusting the liquid flow rate (115) present on the arterial line (108).

15. Blood treatment device by way of extracorporeal circulation according to any one of the preceding claims, **characterized in that** the pre-infusion line (109) is connected to the arterial line (106) as close as possible to the connection of the arterial line to the patient.

16. Blood treatment device by way of extracorporeal circulation according to one of the preceding claims, **characterized in that** the pre-infusion line (109) is operatively associated to a second means for adjusting liquid flow rate (119).

17. Blood treatment device by way of extracorporeal circulation according to one of the preceding claims, **characterized in that** the post-infusion line (112) is operatively associated to a third means for adjusting liquid flow rate (121).

18. Blood treatment device by way of extracorporeal circulation according to one of the preceding claims, **characterized in that** the drain line (110) getting out of the second compartment (104) of the filter (102) includes a fourth means for adjusting liquid flow rate (123) and a first means for measuring liquid flow rate (124).

19. Blood treatment device by way of extracorporeal circulation according to one or more claims 13 to 18, **characterized in that** it comprises a computation and control unit (125) for receiving signals sent by a means for measuring liquid flow rate (124) and for controlling one or more means for adjusting liquid flow rate (115, 119, 121, 123) in order to set a hemofiltration operating mode.

20. Blood treatment device by way of extracorporeal circulation according to one or more claims 13 to 19, **characterized in that** it comprises an inlet line (126) to the second compartment (104) of the filter (102), which connects a dialysis liquid source (127).

21. Blood treatment device by way of extracorporeal circulation according to claim 20, **characterized in that** dialysis liquid contains no calcium ions.

22. Blood treatment device by way of extracorporeal circulation according to claim 20 or 21, **characterized in that** the inlet line (126) comprises a fifth means for adjusting liquid flow rate (128) and a second means for measuring liquid flow rate (129).

23. Blood treatment device by way of extracorporeal circulation according to claim 20, 21 or 22, **characterized in that** it comprises a computation and control unit (125) for receiving signals sent by one or more means for measuring liquid flow rate (124, 129) and for controlling one or more means for adjusting liquid flow rate (115, 119, 121, 123, 128) in order to set a hemofiltration or hemodiafiltration operating mode.

24. Blood treatment device by way of extracorporeal circulation according to one of the claims 13 to 23, **characterized in that** each means for adjusting liquid flow rate (115, 119, 121, 123, 128) comprises a pump or a valve.

25. Blood treatment device by way of extracorporeal circulation according to one of the claims 11 to 20, **characterized in that** each means for measuring liquid flow rate (124, 129) comprises a flow meter.

26. Blood treatment device by way of extracorporeal circulation according to one of the claims 13 to 25, **characterized in that** it comprises a first balance (130) for weighing a first container (131) connected to the pre-infusion line (109) and containing pre-infusion liquid.

27. Blood treatment device by way of extracorporeal circulation according to one of the claims 13 to 26, **characterized in that** it comprises a second balance (132) for weighing the second container (133) connected to the post-infusion line (112) and containing post-infusion liquid.

28. Blood treatment device by way of extracorporeal circulation according to one of the claims 26 or 27, **characterized in that** it comprises a computation and control unit (125) for receiving signals sent by at least one of the two balances (130, 132) and for controlling one or more means for adjusting liquid flow rate (115, 119, 121, 123, 128).

## Patentansprüche

1. Blutbehandlungsvorrichtung durch extrakorporale Zirkulation (101) umfassend:
- einen Filter (102) mit einem ersten (103) und einem zweiten (104) Abteil, die durch eine semipermeable Membran (105) voneinander getrennt sind,
- eine arterielle Leitung (106), die mit dem ersten Abteil (103) des Filters (102) verbunden ist,
- eine venöse Leitung (108) am Ausgang vom ersten Abteil (103) des Filters (102), wobei die venöse Leitung einen Anschluss (139) an dem Patienten aufweist,
- einen Vorinfusionskanal (109) für einen lokalen Antikoagulationsstoff, der mit der arteriellen Leitung (106) verbunden ist,
- einen Dränskanal (110) am Ausgang vom zweiten Abteil (104) des Filters (102),
- einen Luftabscheider (111) an der venösen Leitung (108),
- eine Nachinfusionsleitung (112) für eine Lösung, die den ionischen Gleichgewicht des Blutes mindestens teilweise wiederherstellt, wobei die Nachinfusionsleitung abwärts vom Luftabscheider (111) liegt,
- einen Behälter (133), der mit der Nachinfusionsleitung (112) verbunden ist und eine Lösung enthält, die den ionischen Gleichgewicht des Blutes mindestens teilweise wiederherstellt,
**dadurch gekennzeichnet, daß**:
- sie einen Luftdetektor (113) unmittelbar abwärts von der Nachinfusionsleitung (112) umfasst,
- weder ein Leitungsanschluss noch ein Injektionsbereich sich an der venösen Leitung (108) zwischen dem Luftdetektor (113) und dem Anschluss (139) der venösen Leitung an dem Patienten befindet.

2. Blutbehandlungsvorrichtung durch extrakorporale Zirkulation nach Anspruch 1, **dadurch gekennzeichnet, daß** kein anderer Luftabscheider abwärts vom Luftdetektor (113) eingeschaltet ist.

3. Blutbehandlungsvorrichtung durch extrakorporale Zirkulation nach Anspruch 1 oder 2, **dadurch gekennzeichnet, daß** der benannte Luftabscheider (111) der einzige Luftabscheider in der venösen Leitung (108) ist und keinen Luftdetektor enthält.

4. Blutbehandlungsvorrichtung durch extrakorporale Zirkulation nach einem der vorhergehenden Ansprüche, **dadurch gekennzeichnet, daß** die venöse Leitung (108) einen Kanal umfasst, der ein Verschlussmittel (114) abwärts vom Luftdetektor aufnehmen kann.

5. Blutbehandlungsvorrichtung durch extrakorporale Zirkulation nach Anspruch 4, **dadurch gekennzeichnet, daß** das Verschlussmittel (114) eine Klemme ist.

6. Blutbehandlungsvorrichtung durch extrakorporale Zirkulation nach Anspruch 1, **dadurch gekennzeichnet, daß** der Luftdetektor (113) so nah wie möglich am Anschluss der venösen Leitung (108) an dem Patienten liegt.

7. Blutbehandlungsvorrichtung durch extrakorporale Zirkulation nach einem der vorhergehenden Ansprüche, **dadurch gekennzeichnet, daß** der Luftabscheider (111) einen Blasenfänger umfasst.

8. Blutbehandlungsvorrichtung durch extrakorporale Zirkulation nach einem der vorhergehenden Ansprüche, **dadurch gekennzeichnet, daß** die lokale Koagulationslösung Zitrat-Ionen enthält.

9. Blutbehandlungsvorrichtung durch extrakorporale Zirkulation nach Anspruch 8, **dadurch gekennzeichnet, daß** die lokale Antikoagulationslösung eine Lösung von Trinatriumzitratdihydrat enthält.

10. Blutbehandlungsvorrichtung durch extrakorporale Zirkulation nach Anspruch 8, **dadurch gekennzeichnet, daß** die lokale Antikoagulationslösung eine Lösung von Anticoagulant Citrate Dextrose - als "ACD" bekannt - umfassend Trinatriumzitratdihydrat und Zitronensäuremonohydrat.

11. Blutbehandlungsvorrichtung durch extrakorporale Zirkulation nach einem der vorhergehenden Ansprüche, **dadurch gekennzeichnet, daß** die den ionischen Gleichgewicht des Blutes mindestens teilweise wiederherstellende Lösung Kalzium-Ionen enthält.

12. Blutbehandlungsvorrichtung durch extrakorporale Zirkulation nach Anspruch 8, **dadurch gekennzeichnet, daß** die den ionischen Gleichgewicht des Blutes mindestens teilweise wiederherstellende Lösung eine isotonische Lösung umfassend Kalzium-, Kalium- und Magnesiumchlorid enthält.

13. Blutbehandlungsvorrichtung durch extrakorporale Zirkulation nach einem der vorhergehenden Ansprüche, **dadurch gekennzeichnet, daß** die arterielle Leitung (106) mit einem ersten Mittel zur Einstellung der Blutdurchflussmenge (115) operativ verbunden ist.

14. Blutbehandlungsvorrichtung durch extrakorporale Zirkulation nach irgendeinem der vorhergehenden Ansprüche, **dadurch gekennzeichnet, daß** die Vorinfusionsleitung (109) mit der arteriellen Leitung aufwärts von allen Mitteln zur Einstellung der Flüssigkeitsdurchflussmenge (115) an der arteriellen Leitung (108) verbunden ist.

15. Blutbehandlungsvorrichtung durch extrakorporale Zirkulation nach irgendeinem der vorhergehenden Ansprüche, **dadurch gekennzeichnet, daß** die Vorinfusionsleitung (109) mit der arteriellen Leitung (106) so nah wie möglich am Anschluss der arteriellen Leitung an dem Patienten verbunden ist.

16. Blutbehandlungsvorrichtung durch extrakorporale Zirkulation nach einem der vorhergehenden Ansprüche, **dadurch gekennzeichnet, daß** die Vorinfusionsleitung (109) mit einem zweiten Mittel zur Einstellung der Flüssigkeitsdurchflussmenge (119) operativ verbunden ist.

17. Blutbehandlungsvorrichtung durch extrakorporale Zirkulation nach einem der vorhergehenden Ansprüche, **dadurch gekennzeichnet, daß** die Nachinfusionsleitung (112) mit einem dritten Mittel zur Einstellung der Flüssigkeitsdurchflussmenge (121) operativ verbunden ist.

18. Blutbehandlungsvorrichtung durch extrakorporale Zirkulation nach einem der vorhergehenden Ansprüche, **dadurch gekennzeichnet, daß** die Dränsleitung (110) am Ausgang vom zweiten Abteil (104) des Filters (102) ein viertes Mittel zur Einstellung der Flüssigkeitsdurchflussmenge (123) und ein erstes Mittel zur Messung der Flüssigkeitsdurchflussmenge (124) umfasst.

19. Blutbehandlungsvorrichtung durch extrakorporale Zirkulation nach einem oder mehreren der Ansprüche 13 bis 18, **dadurch gekennzeichnet, daß** sie eine Rechen- und Steuereinheit (125) umfasst zum Empfangen der Signalen, die von einem Mittel zur Messung der Flüssigkeitsdurchflussmenge (124) ausgesandt werden, und zur Steuerung eines oder mehreren Mitteln zur Einstellung der Flüssigkeitsdurchflussmenge (115, 119, 121, 123), um eine Hämofiltrationsbetriebsart anzusteuern.

20. Blutbehandlungsvorrichtung durch extrakorporale Zirkulation nach einem oder mehreren der Ansprüche 13 bis 19, **dadurch gekennzeichnet, daß** sie eine Eingangsleitung (126) zum zweiten Abteil (104) des Filters (102) umfasst, die mit einer Dialyseflüssigkeitsquelle (127) verbindet.

21. Blutbehandlungsvorrichtung durch extrakorporale Zirkulation nach Anspruch 20, **dadurch gekennzeichnet, daß** die Dialyseflüssigkeit keine Kalzium-Ionen enthält.

22. Blutbehandlungsvorrichtung durch extrakorporale Zirkulation nach Anspruch 20 oder 21, **dadurch gekennzeichnet, daß** die Eingangsleitung (126) ein fünftes Mittel zur Einstellung der Flüssigkeitsdurchflussmenge (128) und ein zweites Mittel zur Messung der Flüssigkeitsdurchflussmenge (129) umfasst.

23. Blutbehandlungsvorrichtung durch extrakorporale Zirkulation nach Anspruch 20, 21 oder 22, **dadurch gekennzeichnet, daß** sie eine Rechen- und Steuereinheit (125) umfasst zum Empfangen der Signalen, die von einem oder mehreren Mitteln zur Messung der Flüssigkeitsdurchflussmenge (124, 129) ausgesandt werden, und zur Steuerung eines oder mehreren Mitteln zur Einstellung der Flüssigkeitsdurchflussmenge (115, 119, 121, 123, 128), um eine Hämofiltrations- oder Hämodiafiltrationsbetriebsart anzusteuern.

24. Blutbehandlungsvorrichtung durch extrakorporale Zirkulation nach einem der Ansprüche 13 bis 23, **dadurch gekennzeichnet, daß** jedes Mittel zur Einstellung der Flüssigkeitsdurchflussmenge (115, 119, 121, 123, 128) eine Pumpe oder ein Ventil umfasst.

25. Blutbehandlungsvorrichtung durch extrakorporale Zirkulation nach einem der Ansprüche 11 bis 20, **dadurch gekennzeichnet, daß** jedes Mittel zur Messung der Flüssigkeitsdurchflussmenge (124, 129) einen Durchflussmesser umfasst.

26. Blutbehandlungsvorrichtung durch extrakorporale Zirkulation nach einem der Ansprüche 13 bis 25, **dadurch gekennzeichnet, daß** sie eine erste Waage (130) umfasst zum Wiegen eines ersten Behälters (131), der mit der Vorinfusionsleitung (109) verbunden ist und die Vorinfusionsflüssigkeit enthält.

27. Blutbehandlungsvorrichtung durch extrakorporale Zirkulation nach einem der Ansprüche 13 bis 26, **dadurch gekennzeichnet, daß** sie eine zweite Waage (132) umfasst zum Wiegen des zweiten Behälters (133), der mit der Nachinfusionsleitung (112) verbunden ist und die Nachinfusionsflüssigkeit enthält.

28. Blutbehandlungsvorrichtung durch extrakorporale Zirkulation nach einem der Ansprüche 26 oder 27, **dadurch gekennzeichnet, daß** sie eine Rechen- und Steuereinheit (125) umfasst zum Empfangen der Signalen, die von mindestens einer der beiden Waagen (130, 132) ausgesandt werden, und zur Steuerung eines oder mehreren Mitteln zur Einstellung der Flüssigkeitsdurchflussmenge (115, 119, 121, 123, 128).
